# EUROPEAN PATENT SPECIFICATION

(11) **EP 2 319 938 B1**
(45) Date of publication and mention of the grant of the patent: **01.06.2016**
(21) Application number: 09803072.9
(22) Date of filing: 30.07.2009
(51) Int. Cl.: C12Q 1/68

(54) **METHOD FOR DETECTING CHROMOSOME DEFICIENCIES FOR CONGENITAL ABNORMALITY**
VERFAHREN FÜR DEN NACHWEIS VON CHROMOSOMENDEFIZIENZ FÜR ANGEBORENE ANOMALIEN
PROCÉDÉ DE DÉTECTION D'ANOMALIES CHROMOSOMIQUES ASSOCIÉES À UNE ANORMALITÉ CONGÉNITALE

(30) Priority: 01.08.2008 JP 2008199541
(43) Date of publication of application: 11.05.2011
(73) Proprietor: FUJIFILM Corporation, Tokyo 106-8620 (JP); National University Corporation Tokyo Medical and Dental University, Bunkyo-ku Tokyo 113-8510 (JP)
(72) Inventor: INAZAWA, Johji, Tokyo 113-8510 (JP); IMOTO, Issei, Tokushima 770-0856 (JP); HAYASHI, Shin, Tokyo 113-8510 (JP); AIZU, Yoshinori, Kawagoe-shi Saitama 350-1101 (JP)
(74) Representative: Hoffmann Eitle
(86) International application number: PCT/JP2009/063900
(87) International publication number: WO 2010/013842

(56) References cited:
- JP-A- 2005 304 481
- JP-A- 2008 072 939
- D SANLAVILLE D ET AL: "Molecular karyotyping of two interstitial 10q25 deletions using CGH microarrays", CHROMOSOME RESEARCH, vol. 13 (Suppl. 1), 1 January 2005 (2005-01-01), page 31, XP55029352,
- SHAWN E. MCCANDLESS ET AL: "Adult with an interstitial deletion of chromosome 10 [del(10)(q25.1q25.3)]: Overlap with Coffin-Lowry Syndrome", AMERICAN JOURNAL OF MEDICAL GENETICS, vol. 95, no. 2, 13 November 2000 (2000-11-13), pages 93-98, XP55028987, ISSN: 0148-7299, DOI: 10.1002/1096-8628(20001113)95:2<93::AID-AJ MG1>3.0.CO;2-B
- P. CHIERI ET AL: "Monosomy 10qter due to a balanced maternal translocation: t(10;8)(q23;p23).", CLIN GENET., vol. 24, no. 2, 1 August 1983 (1983-08-01) , pages 147-150, XP55029281,
- SCHRANDER-STUMPEL C ET AL: "The partial monosomy 10q syndrome: report on two patients and review of the developmental data", JOURNAL OF MENTAL DEFICIENCY RESEARCH, NATIONAL SOCIETY FOR MENTALLY HANDICAPPED CHILDREN, LONDON, GB, vol. 35, no. 3, 1 June 1991 (1991-06-01), pages 259-267, XP009160003, ISSN: 0022-264X
- LUKUSA ET AL: "Severe mental retardation-distal arthrogryposis in the upper limbs and complex chromosomal rearrangements resulting from a 10q25-->qter deletion.", CLINICAL GENETICS, vol. 54, no. 3, 1 September 1998 (1998-09-01), pages 224-230, XP55028990, ISSN: 0009-9163
- CARPENTER N J: "MOLECULAR CYTOGENETICS", SEMINARS IN PEDIATRIC NEUROLOGY, SAUNDERS, PHILADELPHIA, PA, US, vol. 8, no. 3, 1 September 2001 (2001-09-01), pages 135-146, XP009016276, ISSN: 1071-9091, DOI: 10.1053/SPEN.2001.26447
- JIE XU ET AL: "Advances in molecular cytogenetics for the evaluation of mental retardation", AMERICAN JOURNAL OF MEDICAL GENETICS, vol. 117C, no. 1, 15 February 2003 (2003-02-15), pages 15-24, XP55029305, ISSN: 0148-7299, DOI: 10.1002/ajmg.c.10016
- VAN DE VOOREN M.J. ET AL.: 'Familial balanced insertion (5;10) and monosomy and trisomy (10) (q24.2 q25.3).' CLIN. GENET. vol. 25, no. 1, 1984, pages 52 - 58, XP008142733
- 'Basic Technology to Establishing Tailor-Made Medicine by Utilizing Genome Information Dai 3 Kai Kokai Symposium Yoshishu, 2007', article JOJI INASAWA ET AL.: 'Ko Seido Genome Array no Kaihatsu to Shikkan Idenshi no Tansaku', pages 19 - 22, XP008142749
- PRESTON R.A. ET AL.: 'gene for Crouzon craniofacial dysostosis maps to the long arm of chromosome 10.' NAT. GENET. vol. 7, no. 2, 1994, pages 149 - 153, XP008142753
- MIYAKO KAWAMURA ET AL.: 'C-gun Senshokutai (10 Ban, 11 Ban, 12 Ban) Kesshitsu no Ekigaku to Rinshozo' ANNUAL REPORT OF HUMAN LIFE SCIENCE, OSAKA CITY UNIVERSITY vol. 38, 1990, pages 461 - 472, XP008142750

## Description

### Technical Field

The present invention relates to a method for detecting a chromosomal deletion for determining whether or not a human subject has a multiple congenital anomaly syndrome accompanying mental retardation, by detecting a hemizygote deletion in a specific region of a human chromosome.

### Background Art

Many congenital anomaly syndrome patients exhibit a deletion in a specific region in chromosomal DNA. Thus far, many congenital anomaly syndromes have been identified in terms of the genomic region having a causal deletion. Among them, some diseases are known to be caused by a hemizygote deletion such as 10qter monosomy (Chieri and Iölster, Clin. Genetics, 1983) . Examples of such congenital anomaly syndromes include Williams syndrome (7q11.2), Smith-Magenis syndrome (17p11.2), Langer-Giedion syndrome (8q24), Wolf-Hirschhorn syndrome (4p16.3), Miller-Dieker syndrome (17p13.3), Prader-Willi and Angelman syndromes (15q11-q13), WAGR syndrome (11p13), Cri du Chat syndrome (5p15.3), Rubinstein-Taybi syndrome (16p13.3), tricho-rhino-phalangeal syndrome (8q24.1), Potoki-Shaffer syndrome (11p11.2), neurofibromatosis I syndrome (17q11), Sotos syndrome (5q35), craniosynostosis syndrome (7p21.1), Kallmann type 1 syndrome (Xp22.3), Kallmann type 2 syndrome (8p11.12), Van der Woude syndrome (1q32-q41), ZFHX1 B deletion syndrome (2q22), blepharophimosis ptosis and epicanthus inversus syndrome (3q23), 1p36 syndrome (1p36), cat eye syndrome (22q11), Alagille syndrome (20p11.23), Diamond-Blackfan syndrome (19q13.2), adrenal hypoplasia congenita (Xp21.2), Coffinlowry syndrome (Xp22.3), DiGeorge syndrome (22q11), Russell-Silver syndrome (7p11.2), and Duchenne Muscular Dystrophy (Xp21.2) (Patent Document 1: "Genomic-DNA-immobilized plate and method for detecting chromosomal aberration and a disease caused thereby by means of the pilate"). Note that the codes enclosed in parentheses represent regions of a chromosome having a hemizygote deletion.

Meanwhile, in some congenital anomaly syndromes, a certain region of a chromosomal DNA fragment has a duplication. For example, in Down syndrome, chromosome 21 is trisomic, and in Pallister-Killian syndrome, the short arm of chromosome 12 is tetrasomic. Pelizaeus Merzbacher disease (dysmyelination) is caused by a duplication in the Xq22 region.

However, many congenital anomaly syndromes are of unknown etiology, such as a multiple system malformation syndrome accompanying mental retardation," which is a target disease whose presence is determined by the present invention. Under such circumstances, it is highly important to thoroughly analyze human genomic DNA to thereby find a deletion or duplication in genomic DNA which is a cause of a disease of unknown etiology. In other words, through identification of a cause of the target disease in a specific genomic DNA fragment, the disease can be correctly or rapidly diagnosed, and an appropriate treatment can be provided. In addition, a causal microstructural aberration in the genome can be identified, leading to a possibility of realizing genetic-level therapy in the future.

### Prior Art Documents

Patent Document 1: Japanese Patent Application Laid-Open (kokai) No. 2005-304481

Non-patent Document 1: Guidebook for Application of Array CGH Diagnosis, edited by Johji INAZAWA, Yoshio MAKITA, and Akira HATA, p. 40-50 and p. 78-81, published on Feb 2008 by Iyaku (Medicine and Drug) Journal Co., Ltd.

### Summary of the Invention

### Problems to be Solved by the Invention

An object of the present invention is to analyze human chromosomes in terms of the presence of a duplication or deletion so as to determine the cause of a multiple system malformation syndrome accompanying mental retardation, and to provide a method for determining whether or not a human subject has the syndrome.

### Means for Solving the Problems

The present inventors have carried out extensive studies in order to find means for effectively identifying genomic aberration which causes a multiple system malformation syndrome accompanying mental retardation (multiple congenital anomaly-mental retardation).

Specifically, pediatricians working in twenty centers registered cases of a multiple congenital anomaly syndrome. From the thus-registered cases, cases which were strongly suspected of having a known congenital anomaly based on the clinical conditions were omitted. Then, the thus-selected cases were analyzed by means of a genome disorder array, whereby diseases caused by a known microstructural aberration or sub-telomere structural aberration were identified. The cases of diseases whose causes could not be identified through the genome disorder array analysis were further investigated by a specialist in pediatric clinical genetics, and the thus-selected cases were thoroughly analyzed by means of an MCG Whole Genome Array-4500. As a result, a plurality of disease cases were identified to have a hemizygote deletion as a genetic aberration in the same region (10q24.31-10q25.1). The present invention has been accomplished on the basis of this finding.

Accordingly, the present invention provides a method for detecting a chromosomal deletion comprising detecting a hemizygote deletion in the region 10q24.31-10q25.1 of a human chromosome (hereinafter may be abbreviated simply as 10q24.31-10q25.1 region or the like) of a human subject as defined in claim 1, to thereby determine whether or not the subject has a multiple congenital anomaly syndrome accompanying mental retardation (hereinafter may be referred to as the detection method of the present invention).

The term "hemizygote deletion" refers to a hetero-type deletion occurring in one chromatid of a chromosome consisting of a pair of chromatids. When a human subject has a homozygote deletion occurring in the 10q24.31-10q25.1 region, the subject is considered to have difficulty in surviving.

The detection method of the present invention includes hybridizing a reference nucleic acid fragment including a part of the 10q24.33 region with a nucleic acid fragment of a specimen, and detecting a signal attributed to a hemizygote deletion of the gene region. Typically, this preferred embodiment is suitably carried out on a substrate on which a nucleic acid fragment including a part of the gene region (hereinafter referred to as "nucleic acid probe") has been immobilized. The substrate is called a "DNA array." Examples of the nucleic acid probe employed in the invention include oligonucleotide, cDNA, BAC (bacterial artificial chromosome) DNA, PAC (phage artificial chromosome) DNA, and YAC (yeast artificial chromosome) DNA. One preferred embodiment of the nucleic acid probe is a gene amplification product obtained through subjecting such nucleic acid fragments to PCR or the like to an unlimited amplification. A specific embodiment of use of such a nucleic acid probe will be described hereinbelow.

The detection method of the present invention may be carried out through the DNA chip method, southern blotting, northern blotting, real-time RT-PCR (polymerase chain reaction), FISH, CGH, or the like.

The specimen which is subjected to the detection method of the present invention is preferably a blood-related specimen, particularly preferably plasma. Alternatively, tissue sections, lymph, sputum, tissue cultures, etc, may be used. Still alternatively, amniotic fluid, cord blood, or villi may be employed as a specimen.

### Effects of the Invention

The present invention enables provision of means for detecting a chromosomal deletion for determining whether or not a human subject has a multiple congenital anomaly syndrome accompanying mental retardation.

### Brief Description of the Drawings

Fig. 1 is a flowchart showing an exemplary scheme of detecting a genomic aberration causing a multiple congenital anomaly syndrome accompanying mental retardation.
Fig. 2A is a photograph showing the results of array CGH analysis of Case 1 using MCG Whole Genome Array-4500 in Example 1, the results being evaluated by relative fluorescence intensity as an index.
Fig. 2B is a photograph showing the results of array CGH analysis of Case 2 using MCG Whole Genome Array-4500 in Example 1, the results being evaluated by relative fluorescence intensity as an index.
Fig. 3 is a photograph showing mapping of genomic aberration regions analyzed by means of MCG Whole Genome Array-4500 in Example 1.
Fig. 4A is a photograph showing the results of analysis of Case 1 using Agilent 244K oligoarray in Example 2, the results being evaluated by relative fluorescence intensity as an index.
Fig. 4B is a photograph showing the results of analysis of Case 2 using Agilent 244K oligoarray in Example 2, the results being evaluated by relative fluorescence intensity as an index.
Fig. 5 is a photograph showing mapping of genomic aberration regions analyzed by means of Agilent 244K oligoarray in Example 2.
Fig. 6 is a photograph showing the results of analysis to detect a chromosomal deletion through fluorescence in situ hybridization using a chromosome preparation obtained from normal human lymphocytes in Example 3.

### Modes for Carrying Out the Invention

### [An embodiment employing a DNA array]

### (1) DNA array

As described above, in carrying out the detection method of the present invention, a DNA array on which a nucleic acid probe such as an oligonucleotide, cDNA, BAC DNA, PAC DNA, or YAC DNA is mounted is preferably employed. Examples of the material of the substrate for the array employed in the invention include glass, plastic material, membrane, or a 3-dimensional array. Among them, generally, a glass substrate such as a glass slide is preferred. The solid substrate (e.g., a glass substrate) is preferably coated, through deposition, with poly-L-lysine, aminosilane, gold-aluminum, or the like.

### (2) WGA-4500 Array

One remarkably powerful embodiment for carrying out the detection method of the present invention may be the array CGH (comparative genomic hybridization) method employing an MCG Whole Genome Array-4500 (may be abbreviated as WGA-4500, see Non-Patent Document 1) on which BAC DNA that completely covers the chromosomes has been mounted. WGA-4500 is a complete genomic array on which 4,523 BAC clones covering the entirety of 22 autosomes and X and Y sex chromosomes, are mounted, and has a mean resolution of about 0.7 Mb. This array is equivalent to about 1/3 of the euchromatin regions of the human chromosomes. In the case where WGA-4500 is employed, when the logarithm (with a base of 2) of the adsorption ratio of the nucleic acid probe containing a part of the 10q24.31-10q25.1 region is -0.3 or less, hemizygote deletion according to the present invention is generally thought to be detected. However, the present invention is not particularly limited to this range.

Based on the above range, more specifically, when the the logarithm (with a base of 2) of the adsorption ratio of RP11-551E2, RP11-416N2, RP11-18I14, RP11-30H12, RP11-16H23, RP11-80B2, RP11-99N20, RP11-541N10, RP11-302K17, RP11-89G15, RP11-68M5, RP11-21N23, RP11-105N15, RP11-302K17, RP11-551E2, RP11-416N2, RP11-18I14, RP11-30H12, RP11-107I14, RP11-108L7, RP-11-91A6, or RP11-37L21 (all being BAC DNA) is -0.3 or less, the hemizygote deletion is thought to be detected.

### (3) Other embodiments of the array

A DNA array in which the number of nucleic acid probes mounted on the array is reduced so as to adapt the aforementioned pattern of hemizygote deletion may also be employed, so long as the DNA array includes at least a nucleic acid probe containing a part of the 10q24.31-10q25.1 region. For example, when BAC DNA fragments are employed as nucleic acid probes, at least one BAC DNA fragment selected from the aforementioned BAC DNA group consisting of the 22 DNA fragments is preferably immobilized on the array. More preferably, one or more BAC DNA fragments corresponding to regions other than the 10q24.31-10q25.1 region are immobilized on the array.

### (4) Unlimited amplification of a target nucleic acid fragment

DNA fragments such as BAC DNA fragments for fabricating a DNA array are generally obtained in so small an amount that a practically large number of genomic DNA-immobilized substrates fails to be produced. Thus, such a DNA fragment is preferably obtained as a gene amplification product. Thus gene amplification step may be referred to as "unlimited amplification." In the unlimited amplification, a BAC DNA fragment or the like is digested with four-base-recognizing enzymes such as *Rsa*I*, Dpn*I*,* and *Hae*III, and the digested product is ligated with an adapter. The adapter is an oligonucleotide having 10 to 30 bases, preferably 15 to 25 bases, and double strands thereof have a complementary nucleotide sequence. After annealing, the 3'-end of the oligonucleotide forming a blunt end must be phosphorylated. Subsequently, PCR is performed by use of a primer having the same sequence as that of the one oligonucleotide of the adapter, whereby the target DNA fragment can be amplified (unlimitedly amplified). Alternatively, an aminated oligonucleotide having 50 to 70 bases, which is typically found in a BAC DNA fragment or the like, may be employed as a detection probe.

The thus-unlimitedly amplified DNA fragments are applied to a substrate preferably at a concentration of 10 pg/µL to 5 µg/µL, more preferably 1 ng/µL to 200 ng/µL. The amount of spotting is preferably 1 nL to 1 µL, more preferably 10 nL to 100 nL. No particular limitation is imposed on the shape and size of each spot to be immobilized onto the substrate. For example, the diameter may be 0.01 to 1 mm, and the shape (as viewed from the top) may be circular or elliptic. No particular limitation is imposed on the thickness of the dried spot, but the thickness is generally 1 to 100 µm. No particular limitation is imposed on the number of the spots, but one substrate preferably has 10 to 50,000 spots, more preferably 100 to 5,000 spots. Each type of DNA fragments is applied in a singular to quadruplicate mode, preferably in a duplicate or triplicate mode.

The dried spots may be obtained through, for example, applying the unlimitedly amplified BAC DNA fragments or the like onto a substrate by means of a dropper to form a plurality of spots, and drying the spots. Examples of the spotter which may be employed in the invention include an ink-jet printer, a pin-array printer, and a bubble-jet (registered trademark) printer. Of these, an ink-jet printer is preferably employed. For example, GENESHOT (registered trademark) (NGK Insulators, Ltd., Nagoya) or the like may be employed.

Through immobilizing the unlimitedly amplified BAC DNA fragments or the like onto a substrate, preferably on a solid substrate, a DNA-fixed substrate of interest can be produced.

### (5) Embodiments of hybridization

### (a) Dual-color fluorescence technique

The hybridization method employing a DNA array and labeled nucleic acid fragments [i.e., CGH (comparative genomic hybridization) method] may be carried out through, for example, the dual-color fluorescence technique.

In one embodiment of the dual-color fluorescence technique, labeled target nucleic acid fragments originating from two different samples are applied to a sheet of DNA array or one hybridization region. The labeled target nucleic acid fragments are bonded to different kinds of labeling compounds. A labeled target nucleic acid fragment is prepared by labeling a target nucleic acid fragment originating from a normal specimen, and another labeled target nucleic acid fragment is prepared by labeling a target nucleic acid fragment originating from a specimen of a patient. The two labeled target nucleic acid fragments are mixed together and hybridized with the nucleic acid probes immobilized on a sheet of CGH array. The ratio of one adsorbed target fragment to the other adsorbed fragment is calculated. For example, if the target fragments have been labeled with fluorescent labeling agents, the ratio is calculated from fluorescence intensities.

### (b) Labelling

In the present invention, the term "labeling" refers to bonding of a detectable substance to a target nucleic acid fragment. Any substance may be incorporated into the target nucleic acid fragments of the present invention, so long as the substance is detectable. Examples of the labeling substance which may be employed in the invention include a fluorescence substance, an inorganic compound, a protein (e.g., an enzyme-labeled antibody used in enzyme-linked immunosorbent assay (ELISA) or the like), a radioisotope, and fluorescence resonance energy transfer (FRET).

No particular limitation is imposed on the fluorescent substance which is employed as a labeling agent. Examples of the fluorescent substance which may be used in the invention include fluorescein isothiocyanate (FITC), Cy3, Cy5, Cy7, green fluorescent protein (GFP), blue fluorescent protein (BFP), yellow fluorescent protein (YFP), red fluorescent protein (RFP), Alexa, acridine, DAPI, ethidium bromide, SYBR Green, Texas Red, a rare earth fluorescent labeling agent [4,4' -bis(1'',1'', 1",2",2",3",3"-heptafluoro-4",6"-hexanedion-6"-yl)-chlorosulfo-o-terphenyl (BHHCT)], acridine orange, TAMRA, and ROX.

No particular limitation is imposed on the inorganic compound which is used as a labeling agent, and examples include a quantum dot formed of a semiconducting inorganic material. Specific examples include nanoparticles of silica, CdTe, ZnSe, and CdSe. The wavelengths of the fluorescences emitted by these nano-particulate inorganic materials may be shifted by modifying the corresponding particle sizes thereof. When the particle size (diameter) is 2 nm, 3 nm, 4 nm, and 5 nm, the color of the fluorescence assumes blue, green, yellow, and red, respectively- Thus, such fluorescence can be detected, and the presence of the corresponding particles can also be detected. For example, the particles may be detected by means of an atomic force microscope (AFM).

The labeling agent may also be digoxigenin (DIG), biotin, etc. In the case where biotin is used, avidin is caused to bind to biotin which has been bound to a target nucleic acid fragment, and a biotin-bound alkaline phosphatase is caused to bind to avidin. Through addition of nitroblue-tetrazolium and 5-bromo-4-chloro-3-indolylphosphoric acid serving as substrates for the alkaline phosphatase, purple coloring occurs, and the coloring may be employed for the detection.

In an alternative embodiment, non-enzymatic labeling may be performed. For example, a ULS™ array CGH labeling kit (product of Kreatech Biotechnology BV) or the like may be used.

### (c) Purification of target nucleic acid fragments

In the present invention, a target nucleic acid fragment must be purified in the preparation thereof from a specimen. During the below-mentioned labeling step, various side reactions occur, and cell lysis products such as protein and lipid considerably affect the background noise. Thus, unless purification is performed, the performance and reliability of the hybridization test employing a nucleic acid microarray or the like is considerably impaired.

As used herein, the term "purification" is a synonym for extraction, separation, or fractionation. Examples of the purification means which may be employed in the invention include a technique employing a cartridge in which a nucleic acid-adsorbing membrane such as silica or a cellulose derivative is incorporated; precipitation in ethanol or isopropanol; phenol-chloroform extraction; a technique employing a solid-phase extraction cartridge which contains an ion-exchange resin, a silica carrier onto which a hydrophobic substituent (e.g., an octadecyl group) has been bound, or a resin exhibiting a size exclusion effect; and chromatographic techniques. The purification may also be performed through electrophoresis. Also, in the present invention, solvent replacement is defined as a purification step in a broad sense.

In the present invention, the purification step may also be performed twice in the preparation of target nucleic acid fragments from target cells. In the present invention, the first purification step (i.e., recovering a target nucleic acid fragment from a target cell) may be performed through a technique employing a cartridge in which a nucleic acid-adsorbing membrane such as silica or a cellulose derivative is incorporated; precipitation in ethanol or isopropanol; phenol-chloroform extraction; or the like. Among these purification means, the product "QuickGene Series" (product of FUJIFILM Corporation) - a cartridge in which a nucleic acid-adsorbing porous membrane prepared through saponification of triacetyl cellulose is deposited - is preferably employed for purification, since target nucleic acid fragments can be semi-automatically prepared by means of an inexpensive apparatus, through use of the product "QuickGene Series."

A further purification step may be added. Specifically, the purification step is performed in order to enhance the concentration and purity of the prepared target nucleic acid fragment. In the first purification step, when phenol-chloroform extraction or any of various precipitation techniques is employed, the purification performance is generally inferior to that attained though the column technique, and the low performance adversely affects a subsequent step. The second purification step may be performed through a technique employing a cartridge in which a nucleic acid-adsorbing membrane such as silica is incorporated; precipitation in ethanol or isopropanol; a technique employing a solid-phase extraction cartridge which contains an ion-exchange resin, a silica carrier onto which a hydrophobic substituent (e.g., an octadecyl group) has been bound, or a resin exhibiting a size exclusion effect; or chromatographic techniques. Among these purification means, a QucikGene SP kit (product of FUJIFILM Corporation) is most preferably employed for purification.

The nucleic acid-adsorbing porous membrane of the QucikGene SP kit is very thin as compared with a silica-based nucleic acid-adsorbing porous membrane. Therefore, the membrane is suitable for extraction of nucleic acid from a sample of small amount, and enables recovery of a target nucleic acid fragment at very high concentration as compared with other purification means. In addition, as compared with precipitation in ethanol or isopropanol, which also enables purification of nucleic acid from a sample of small amount, the membrane of the QucikGene SP kit is able to provide a target nucleic acid fragment at higher purity, and thus is suitable for recovering a target nucleic acid fragment in a small amount and at high concentration.

### [Other embodiments of detection]

### (1) Chromosome banding

If a multiple system malformation syndrome of unknown cause accompanying mental retardation accompanies an aberration in a specific region of the human genome, the syndrome can be detected through a chromosome banding technique. Particularly when the 10q24.31-10q25.1 region has a hemizygote deletion of about 10 Mb, the syndrome can be detected through a chromosome banding technique such as G-banding. Specifically, the syndrome can be detected by observing an aberration in banding state of the region of the present invention (i.e., presence of disappearance of a chromosome band including the 10q24.31-10q25.1 region)-(2) Detection by the FISH technique

When the genomic aberration has a small size of some Mb or less, the syndrome can be detected based on a signal generated by hybridization of nucleic acid fragments through, for example, the FISH (fluorescence in situ hybridization) technique (Yasui, K., Imoto, I., Fukuda, Y., Pimkhaokham, A., Yang, Z. Q., Naruto, T., Shimada, Y., Nakamura, Y., and Inazawa, J., "Identification of target genes within an amplicon at 14q12-q13 in esophageal squamous cell carcinoma," Genes Chromosomes Cancer, 32, 112-118, 2001). In this case, FISH probes of the 10q24.31-10q25.1 region are prepared, and hybridized with chromosomes derived from a patient. Through observation of a decrease in number of signals, a hemizygote deletion can be detected.

### (3) Southern blotting technique

In the southern blotting technique, the genomic DNA obtained from a specimen is digested by restriction enzymes, and the digested products are subjected to gel electrophoresis. The products are immobilized on a nitrocellulose membrane, and the DNA fragments are hybridized with labeled DNA fragments present in the 10q24.32-q25.1 region, whereby a target gene contained in the specimen is detected. A hemizygote deletion can be identified in the case where the amount of detection (band concentration) of the patient's specimen is smaller than that of a normal specimen, and a new band appears.

### (4) Northern blotting technique

In the northern blotting technique, all RNA fragments recovered from a specimen are subjected to electrophoresis, and the products are immobilized on a membrane in a manner similar to that employed in southern blotting, and the RNA fragments are hybridized with labeled DNA fragments present in the 10q24.32-q25.1 region, whereby a target gene contained in the specimen is detected. A hemizygote deletion may be identified in the 10q24.32-q25.1 region in the case where the amount of detection (band concentration) of the patient's specimen is smaller than that of a normal specimen.

### (5) Real-time RT-PCR technique

In the real-time RT-PCR technique, at least one primer corresponding to a transcription product of the 10q24.32-q25.1 region of the DNA fragment present in the specimen is provided, and the primer is subjected to reverse transcription. The reverse transcription product is then subjected to a gene amplification step. The target gene is detected on the basis of formation of the corresponding amplicon or the amount of amplicon. A hemizygote deletion may be identified in the case where the amount of DNA amplicon of the patient's specimen is smaller than that of a normal specimen.

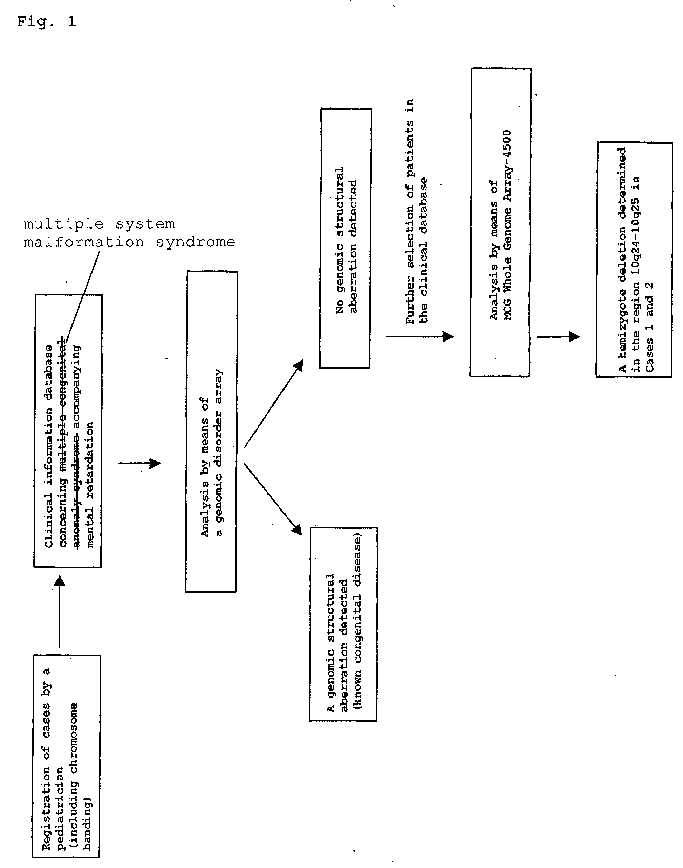

Fig. 1 is a scheme of identification of a genomic aberration according to the present invention. More specific features of the identification procedure are disclosed in the Examples below.

Examples

The present invention will next be described in more detail by way of examples.

### (A) MCG Whole Genome Array-4500

As described above, in carrying out the detection method of the present invention, MCG Whole Genome Array-4500 is used as means for detecting the correlation between a hemizygote deletion in the 10q24.31-10q25.1 region present in the human chromosomes and the multiple system malformation syndrome accompanying mental retardation. Although this detection array is a known product (Non-Patent Document 1), the production step will be briefly described.

Through searching "The National Center for Biotechnology Information (NCBI)" and the genome database website and the BLAST results of the selected DNA provided by University of California, Santa Cruz, 4,523 BAC/PAC clones present in the euchromatin region of the human genome were selected.

BAC DNA fragments and PAC DNA fragments were prepared from the thus-selected clones; the fragments were digested with *Dpn*I*, Rsa*I*,* and *Hae*III, and ligated with an oligonucleotide for adapter synthesis. Subsequently, PCR was twice performed by use of a primer having a sequence of the adapter. This process is called "unlimited amplification," and the thus-obtained DNA fragments are defined as unlimitedly amplified DNA fragments. The unlimitedly amplified DNA fragments were applied onto the array twice by means of an ink-jet-type spotter (GENESHOT (registered trademark), NGK Insulators, Ltd., Nagoya), to thereby produce a high-density CGH array of interest (MCG Whole Genome Array-4500).

### (B) Identification of a causal region in the human chromosomes

### (1) Clinical features of two patients with a multiple system malformation syndrome accompanying mental retardation

In the Examples, in order to identify a region in the human chromosomes corresponding to the multiple system malformation syndrome accompanying mental retardation, two specimen-donors (i.e., two infant patients with undetermined congenital abnormality accompanying mental retardation (Cases 1 and 2)) were investigated, and the disclosable clinical features of the patients are as follows.

Case 1 (male infant) was born with a body weight of 2,830 g without undergoing asphyxia at birth. At the age of 4 years and 0 month, he was diagnosed to have severe mental retardation, macrocephaly (+2SD), exotropia, and abnormalities in the face. More specifically, slight megacephaly and prominent forehead were observed in the head and neck portion and the face; severe esotropia was observed in the eyeballs; and upturned nose and saddle nose were observed in the nose. Regarding development and neuro-conditions, severe mental development retardation was observed; acquirement of the skill of head holding-up and sitting alone required five months and 24 months, respectively; no bipedal walking or utterance was observed; and no seizure was observed as a neuro-condition. Heavy autism and hypomyotonia were observed.

Case 2 (male infant) was born with a weight of 2,458 g and found to have cardiovascular diseases of atrial septal defect, ventricular septal defect (VSD), and patent ductus arteriosus (PDA). At the age of 3 years and 8 months, he was diagnosed to have cheilo/palatoschisis, hypacusis (100 dB), microphthalamia, short fingers, peculiar facial appearances (thick eyebrows, wide radix nasi, epicanthus, and wide nose), a single-strand broken line in the fifth finger, and a backward leant posture. More specifically, regarding the head and neck portion and the face, ocular hypertelorism, epicanthus, and a small right eye ball were observed in the eye area; and flat dorsum nasi, upturned nose, slight saddle nose were observed in the nose. In the mouth area, cheilo/palatoschisis was observed. Regarding the trunk, cryptorchism was observed in the pudendum. Regarding extremities, a short and inflected little finger was observed in the fingers. Regarding development and neuro-conditions, severe mental development retardation was observed (development quanitiy (DQ): 20 to 30); sitting alone could not be acquired; and no seizure was observed as a neuro-condition. Hypomyotonia was observed.

### (2) Analysis by means of a DNA array [Example 1]

### (a) Results of analysis by means of a genome disorder array

A genome disorder array is a substrate on which there have been immobilized genomic DNA fragments (BAC clones) included in a region of human genomic DNA where a deletion or amplification attributable to congenital abnormality is observed.

The analysis by means of a genome disorder array enables detection of the following: Williams syndrome, Smith-Magenis syndrome, Down syndrome, Langer-Giedion syndrome, Wolf-Hirschhorn syndrome, Miller-Dieker syndrome, Prader Willi and Angelman syndrome, WAGR syndrome, Cri du Chat syndrome, Pallister-Killian syndrome, Rubinstein-Taybi syndrome, tricho-rhino-phalangeal syndrome, Potoki-Shaffer syndrome, neurofibromatosis I syndrome, Sotos syndrome, craniosynostosis syndrome, Kallmann type 2 syndrome, Kallmann type 1 syndrome, Van der Woude syndrome, ZFHX1 B deletion syndrome, blepharophimosis and epicanthus syndrome, 1p36 syndrome, cat eye syndrome, Alagille syndrome, Diamond-Blackfan syndrome, adrenal hypoplasia congenita syndrome, steroid sulfatase syndrome, Digeorge syndrome, Russell-Silver syndrome, Duchenne Muscular Dystrophy, Pelizaeus Merzbacher Disease, and sub-telomere abnormality.

DNA fragments were extracted from each of the infant patients with undetermined multiple system malformation syndrome syndrome accompanying mental retardation (Cases 1 and 2), and analyzed through the array CGH method by means of a genome disorder array in a typical manner. In the analysis, no causal structural aberration was detected in the genome.

### (b) Analysis by means of MCG Whole Genome Array-4500

DNA fragments were prepared from the blood of each patient (Case 1 or 2), and the structural aberration in the genome was investigated through the array CGH method by means of the aforementioned MCG Whole Genome Array-4500 in the following manner.

DNA fragments derived from each of the infant patients with undetermined multiple system malformation syndrome accompanying mental retardation (Cases 1 and 2) were Cy3-labeled by means of BioPrime DNA labeling System (Invitrogen, USA), and DNA fragments derived from a healthy subject were Cy5-labeled. Both DNA samples (each 50 µL), Cot-1 DNA (Invitrogen, USA) (250 µL), 3M NaOAc (Sigma, USA) (35 µL), and 100% ethanol (875 µL) were mixed, and the mixture was cooled at -80°C for 10 minutes and centrifuged at 4°C and 15,000 rpm for 30 minutes.

Separately, the MCG Whole Genome Array-4500 was immersed in boiling sterile water for two minutes and then sequentially in 70%, 85%, and 100% cold ethanol for two minutes for dehydration, followed by drying. The thus-treated array was maintained at 42°C. The thus-treated MCG Whole Genome Array-4500 was placed in Hybri-Master HS-300 (ALOKA Co., Ltd., Tokyo), and a preliminary hybridization liquid (MM 40 µL, yeast tRNA 6 µL, and 20% SDS 12 µL) was added dropwise to the array, whereby preliminary hybridization was performed at 42°C for 10 minutes. Subsequently, another hybridization liquid (MM 80 µL, yeast tRNA 12 µL, and 20% SDS 24 µL) in which each DNA sample was dissolved was added dropwise, whereby hybridization was performed at 42°C for 48 to 72 hours. As used herein, MM refers to a master mixture, which is prepared by mixing formamide (5 mL), dextran sulfate (1 g), and 20xSSC (1 mL) and sufficiently dissolving the mixture with distilled water so as to adjust the volume to 7 mL.

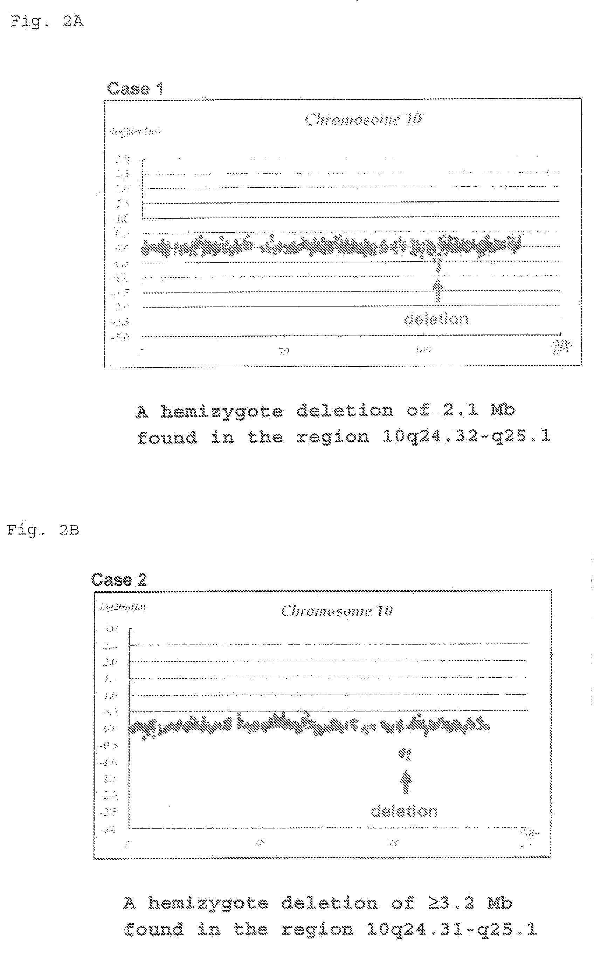

Then, the array was sequentially washed with 2xSSC (maintained at 50°C) for one minute and 10 minutes, 50% formamide/2×SSC (pH: 7.0, maintained at 50°C) for 10 minutes, and 1×SSC (maintained at 42°C) for 10 minutes. Thereafter, the microarray was sufficiently dried and subjected to fluorescence scanning by means of GenePix 4000B (Amersham Biosciences, USA) (Cy3 fluorescence by 532 nm laser beam, and Cy5 fluorescence by 635 nm laser beam). Since Cy3 fluorescence and the Cy5 fluorescence have energies considerably different from each other, the total intensity obtained from the all spots in the case of Cy3 fluorescence was normalized to that in the case of Cy5 fluorescence, and the ratio Cy3/Cy5 at each spot was obtained. The ratio was converted to its logarithm value; i.e., log₂(ratio). In Fig. 2, the vertical axis represents log₂(ratio), and the horizontal axis represents the location in the human chromosome 10 (Mb); i.e., a range of about 144 MB from the top of the short arm to the end of the long arm. Fig. 2 consists of Fig. 2A (Case 1) and Fig. 2B (Case 2).

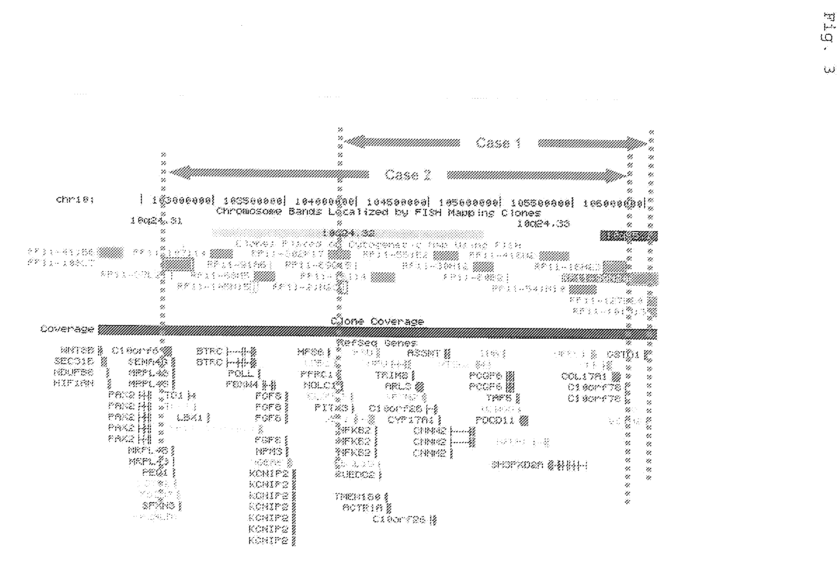

As a result, a hemizygote deletion of 2.1 Mb was detected in the region of 10q24.32-q25.1 of chromosome 10 of the Case 1 patient, and a hemizygote deletion of ≥3.2 Mb was detected in the region of 10q24.31-q25.1 of the Case 2 patient. The BAC DNA fragments found in the deletion region were RP11-551E2, RP11-416N2, RP11-18I14, RP11-30H12, RP11-16H23, RP11-80B2, RP11-99N20, RP11-541N10, RP11-302K17, RP11-89G15, and RP11-68M5 in Case 1, and RP11-107I14, RP11-108L7, RP11-91A6, RP11-37L21, RP11-68M5, RP11-21N23, RP11-551E2, RP11-416N2, RP11-18I14, RP11-30H12, RP11-16H23, RP11-80B2, RP11-541N10, RP11-302K17, RP11-89G15, RP11-68M5, RP11-105N15, RP11-302K17, RP11-551E2, RP11-416N2, RP11-18I14, and RP11-30H12 in Case 2. Through comparison of Case 1 with Case 2, BAC DNA fragments RP11-551E2, RP11-416N2, RP11-18I14, RP11-30H12, RP11-16H23, RP11-80B2, RP11-541N10, RP11-302K17, RP11-89G15, RP11-21N23, and RP11-99N20 were found to be present in the both cases. Fig. 3 shows a map of the hemizygote deletion regions found in Cases 1 and 2.

The analysis performed in Example 1 has revealed that a multiple system malformation syndrome accompanying mental retardation can be determined by the presence of a hemizygote deletion in the 10q24.32-q25.1 region. Example 1 has also revealed that detection of the hemizygote deletion by means of MCG Whole Genome Array-4500 is essential means for multiple system malformation syndrome determining a multiple system malformation syndrome accompanying mental retardation.

According to the analytical results, when means for detecting a hemizygote deletion in the 10q24.32-q25.1 region is employed, the detection method of the present invention can be surely carried out. In other words, a hemizygote deletion in the 10q24.32-q25.1 region is detected through a known gene analysis technique such as the DNA chip technique, southern blotting, northern blotting, real-time RT-PCR, the FISH technique, or the CGH technique, wherein the genetic deletion (aberration) is detected as a target, and a multiple system malformation syndrome accompanying mental retardation can be determined.

Next, an analysis through the CGH technique employing a gene detection substrate other than MCG Whole Genome Array-4500 (Example 2) and an analysis based on the FISH technique (Example 3) will be described. Needless to say, techniques should not be construed as limiting the scope of the invention thereto. In other words, so long as the detection sensitivity allows the aforementioned genetic deletion (aberration) to be identified, the present invention may be carried out through a wide range of techniques, such as techniques in which a signal generated by hybridization between the genes of a specimen and nucleic acid fragments (such as oligonucleotide, cDNA, BAC DNA, PAC DNA, or YAC DNA) corresponding to the 10q24.32-q25.1 region is detected (e.g., the DNA chip technique, southern blotting, northern blotting, real-time RT-PCR, the FISH technique, or the CGH) and techniques which are based on a detection principle other than hybridization, such as chromosome banding (e.g., G-banding).

### [Example 2]

### Analysis by means of Agilent 244K oligoarray (Agilent, USA)

Each (0.2 µg) of the DNA samples prepared from the Case 1 patient and the Case 2 patient was analyzed through the CGH technique employing Olig-aCGH Microarray Type 244K (Agilent). Since the array of type 244K covers more than 236,000 coding and non-coding regions of the human genome, the human genome can be thoroughly analyzed. The mean resolution of the probes is 6.4 Kb. The analysis was carried out through the following general procedure according to a protocol of Agilent.

Each (2.0 µg) of the DNA fragment samples of the Case 1 patient and the Case 2 patient and the DNA fragment sample of a healthy subject was digested with two restriction enzymes (*Alu*I and *Rsa*I) and labeled through the nick translation technique employing an Exo-Klenow fragment. Labeling was performed with a fluorescence dye Cy5 in the case of the DNA fragments derived from a healthy subject, and with a fluorescence dye Cy3 in the case of the DNA fragments derived from a patient. The thus-labeled DNA fragments were purified by means of Microcon YM-30 filter unit (Millipore).

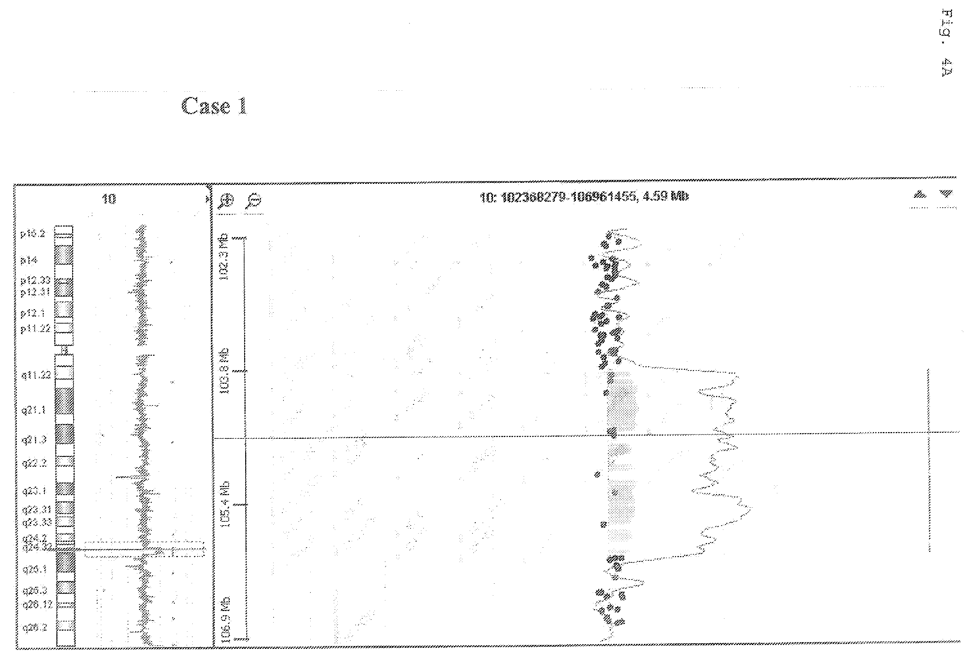

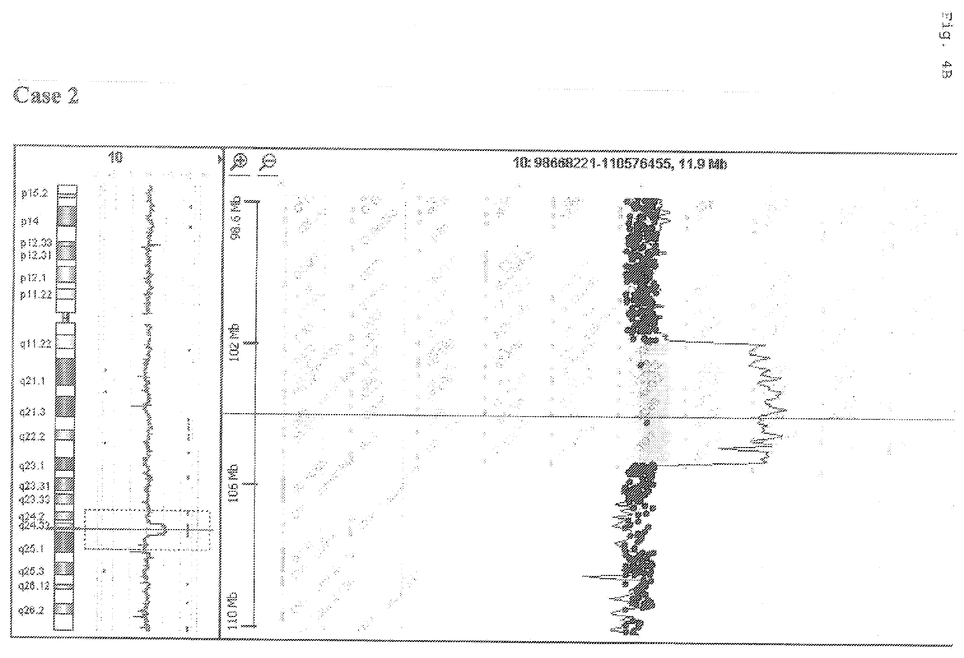

The Cy3-labeled and Cy5-labeled DNA samples (each 158 µL), 1.0-mg/mL Human Cot1 DNA (50 µL), 10×blocking agent (52 µL), and 2×hybridization buffer (260 µL) were mixed and heated at 95°C, and the mixture was cooled to 37°C. The thus-obtained solution (490 µL) was placed on a gasket slide on which Agilent 244K Oligonucleotide Array had been immobilized, and the system was fitted with metal fittings. Hybridization was performed at 65°C for 40 hours. The array was washed in buffer 1 at room temperature for five minutes and then in buffer 2 maintained at 37°C for one minute under stirring. This microarray was sufficiently dried and subjected to fluorescence scanning by means of GenePix 4000B (Amersham Biosciences, USA) (Cy3 fluorescence by 532 nm laser beam, and Cy5 fluorescence by 635 nm laser beam). Since Cy3 fluorescence and the Cy5 fluorescence have energies considerably different from each other, the total intensity obtained from the all spots in the case of Cy3 fluorescence was normalized to that in the case of Cy5 fluorescence, and the ratio Cy3/Cy5 at each spot and its logarithm value; i.e., log₂(ratio), were obtained. Fig. 4 shows the results. In Fig. 4, the vertical axis represents -log₂(ratio), and the horizontal axis represents the location in the human chromosome 10 (Mb); more specifically, in a region of 4.59 Mb (Mb: megabases, precisely 4,593,176 bases) ranging from 102.3 Mb (precisely 102,368,279 bases) to 106.9 Mb (precisely 106,961,455 bases) (in Case 1) and in a region of 11.9 Mb (precisely 11,908,234 bases) ranging from 98.6 Mb (precisely 98,668,221 bases) to 110 Mb (precisely 110,576,455 bases) (in Case 2). In Fig. 4, a chart showing the short arm and long arm of the chromosome 10 and a cGH analysis chart of the entire chromosome 10 obtained through the Agilent 244K Oligoarray analysis are attached to the left side of each graph. The gray lines and broken lines indicate the location of the genes shown to the right thereof in the chromosome 10. Fig. 4 consists of Fig. 4A (Case 1) and Fig. 4B (Case 2).

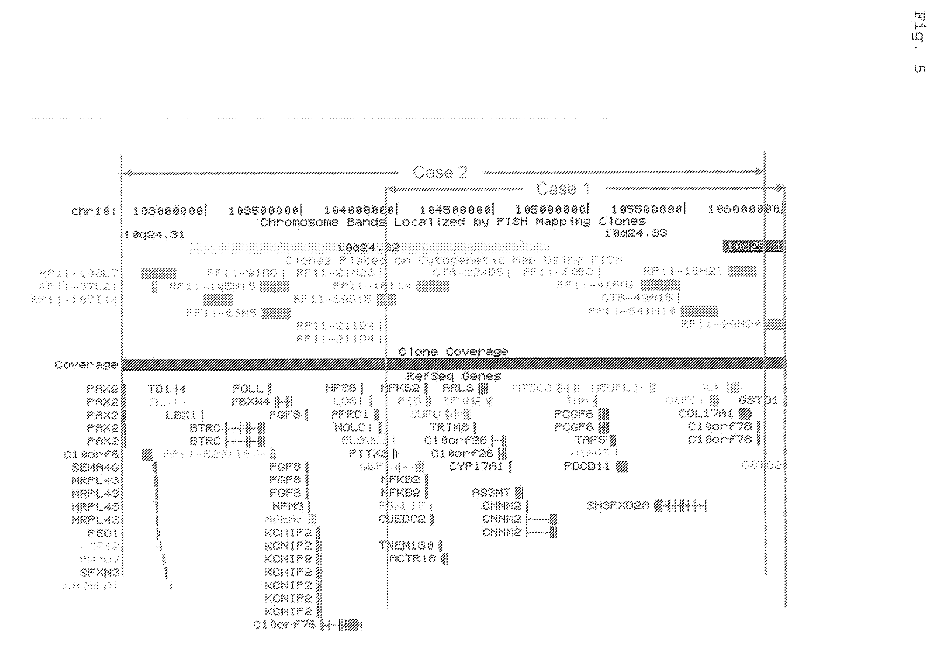

In Example 2, the same results as obtained in Example 1 (b) were obtained, and the size of a deletion could be precisely determined. Specifically, a hemizygote deletion of 2.1 Mb was detected in the region of 10q24.32-q25.1 of chromosome 10 of the Case 1 patient, and a hemizygote deletion of 3.3 Mb was detected in the region of 10q24.31-q25.1 of the Case 2 patient. Fig. 5 shows a map of the hemizygote deletion regions found in Cases 1 and 2.

### [Example 3]

### Analysis through the FISH technique

The fact that a patient of a multiple system malformation syndrome malformation syndrome accompanying mental retardation has a hemizygote deletion in the 10q24.31-10q25.1 region of a chromosome was confirmed through the FISH technique. Firstly, lymphocyte samples were prepared from the blood of the aforementioned two patients, whereby metaphase chromosomes were produced. Specifically, human lymphocytes were cultured with 12.5-µg/mL phytohemagglutinin for three days. Then, 0.025-µg/mL colcemid was added to the culture, and the mixture was further cultured for several hours. The supernatant was removed, and 0.075M KC1 hypotonic solution was added to the residue. The mixture was allowed to stand for 30 minutes. Subsequently, the supernatant was removed, and the lymphocytes were fixed with Carnoy's fixative. The chromosomes were developed on a glass slide, to thereby produce metaphase chromosomes.

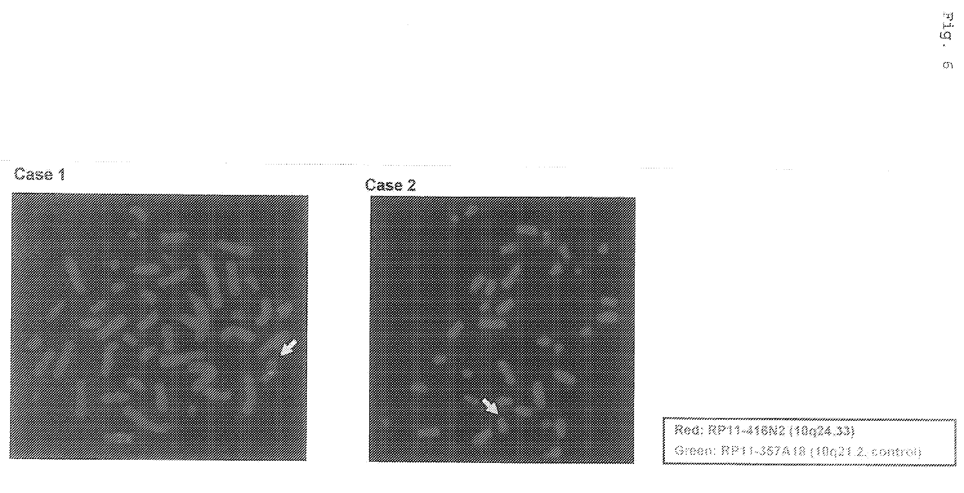

BAC DNA fragments (RP11-416N2) contained in a hemizygote deletion region (10q24.33) were allowed to react at 16°C overnight by means of a nick translation kit, to thereby incorporate digoxigenin-11-dUTP thereinto. In a similar manner, BAC DNA fragments (RP11-357A18) (control) contained in the 10q21.2 region having no deletion in the chromosome of the patients were allowed to react, to thereby incorporate biotin-16-dUTP thereinto. Subsequently, both labeled BAC DNA fragments were heated at 75°C for ten minutes and then cooled with ice. Hybridization was performed on the metaphase chromosome overnight at 37°C. Free labelled BAC DNA fragments which had not been subjected to hybridization were sequentially washed with 50% formamide/2×SSC (pH: 7.0) at 37°C for 15 minutes, with 2xSSC at room temperature for 15 minutes, and with 1×SSC at room temperature for 15 minutes. For fluorescent labeling, 0.02-mg/mL avidin-FITC and 1.2-µg/mL anti-digoxigenin-rhodamine were added thereto, and the mixture was allowed to react on the metaphase chromosomes at 37°C for one hour. Thereafter, the reaction product was sequentially washed with 4xSSC at room temperature for ten minutes, with 4xSSC containing 0.05% Triton-X-100 at room temperature for ten minutes, and with 4xSSC at room temperature for ten minutes. The metaphase slide was dried through centrifugation, and a drop of 125-ng/mL 4',6-diamino-2-phenylindol (DAPI) was added thereto. A glass cover was put on the slide, and the chromosomes were observed under a microscope. As shown in the obtained photographs, two chromosomes of the long arm of the chromosome 10 were stained green (fluorescent) by control BAC DNA fragments (RP11-357A18) in Cases 1 and 2, and only one chromosome of the long arm of the chromosome 10 was stained red (fluorescent) by BAC DNA fragments (RP11-416N2) contained in the deletion region 10q24.33 (Fig. 6). Therefore, Case 1 patient and Case 2 patient were found to have a hemizygote deletion in the 10q24.33 region.

### Industrial Applicability

The inventors have found that the two cases of a multiple system malformation syndrome accompanying mental retardation were caused by a hemizygote deletion in the same region (10q24-10q25) of the chromosome. On the basis of this finding, checking the presence of an aberration in the 10q24-10q25 region of the chromosome enables definite diagnosis of a multiple system malformation syndrome accompanying mental retardation.

## Claims

1. A method for detecting a chromosomal deletion comprising detecting a hemizygote deletion in the region 10q24.33 of a human chromosome of a human subject by hybridizing a reference nucleic acid fragment including a part of the 10q24.33 region with a nucleic acid fragment of a specimen of the human subject, and detecting a signal attributed to the hemizygote deletion of the 10q24.33 region, to thereby determine whether or not the subject has a multiple system malformation syndrome accompanying mental retardation.

2. The method for detecting a chromosomal deletion according to claim 1 , wherein hybridizing the reference nucleic acid fragment including a part of the 10q24.33 region with the nucleic acid fragment of a specimen is carried out on a substrate on which the reference nucleic acid fragment including a part of the 10q24.33 region has been immobilized.

3. The method for detecting a chromosomal deletion according to claim 1 or 2, wherein the reference nucleic acid fragment including a part of the 10q24.33 region is oligonucleotide, cDNA, BAC DNA, PAC DNA, or YAC DNA.

4. The method for detecting a chromosomal deletion according to claim 3, wherein the reference nucleic acid fragment including part of the 10q24.33 region is the BAC DNA RP11-416N2.

5. The method for detecting a chromosomal deletion according to any one of claims 1 to 4, wherein the hemizygote deletion in the 10q24.33 region is detected by means of the DNA chip technique, southern blotting, northern blotting, real-time RT-PCR, the FISH technique, or the CGH technique.

## Patentansprüche

1. Verfahren zum Detektieren einer chromosomalen Deletion, umfassend Detektieren einer hemizygoten Deletion in der Region 10q24.33 eines menschlichen Chromosoms eines menschlichen Subjekts durch Hybridisieren eines Referenz-Nukleinsäurefragments einschließlich eines Teils der 10q24.33-Region mit einem Nukleinsäurefragment einer Probe des menschlichen Subjekts, und Detektieren eines der hemizygoten Deletion der 10q24.33-Region zugeschriebenen Signals, um dadurch zu ermitteln, ob das Subjekt ein geistige Behinderung begleitendes multiples Systemmissbildungssyndrom (a multiple system malformation syndrome accompanying mental retardation) hat oder nicht.

2. Verfahren zum Detektieren einer chromosomalen Deletion gemäß Anspruch 1, wobei Hybridisieren des Referenz-Nukleinsäurefragments einschließlich eines Teils der 10q24.33-Region mit dem Nukleinsäurefragment einer Probe auf einem Substrat durchgeführt wird, auf dem das Referenz-Nukleinsäurefragment einschließlich eines Teils der 10q24.33-Region immobilisiert worden ist.

3. Verfahren zum Detektieren einer chromosomalen Deletion gemäß Anspruch 1 oder 2, wobei das Referenz-Nukleinsäurefragment einschließlich eines Teils der 10q24.33-Region Oligonukleotid, cDNA, BAC-DNA, PAC-DNA, oder YAC-DNA ist.

4. Verfahren zum Detektieren einer chromosomalen Deletion gemäß Anspruch 3, wobei das Referenz-Nukleinsäurefragment einschließlich eines Teils der 10q24.33-Region die BAC-DNA RP11-416N2 ist.

5. Verfahren zum Detektieren einer chromosomalen Deletion gemäß einem der Ansprüche 1 bis 4, wobei die hemizygote Deletion in der 10q24.33-Region mittels der DNA-Chip-Methode, Southern Blot, Northern Blot, real-time RT-PCR, der FISH-Methode, oder der CGH-Methode detektiert wird.

## Revendications

1. Procédé de détection d'une délétion chromosomique comprenant la détection d'une délétion hémizygote dans la région 10q24.33 d'un chromosome humain d'un sujet humain par l'hybridation d'un fragment d'acide nucléique de référence comprenant une partie de la région 10q24.33 avec un fragment d'acide nucléique d'un échantillon du sujet humain, et la détection d'un signal attribué à la délétion hémizygote de la région 10q24.33, pour ainsi déterminer si le sujet présente ou non un syndrome de malformation multisystémique s'accompagnant d'un retard mental.

2. Procédé de détection d'une délétion chromosomique selon la revendication 1, dans lequel l'hybridation du fragment d'acide nucléique de référence comprenant une partie de la région 10q24.33 avec le fragment d'acide nucléique d'un échantillon est réalisée sur un substrat sur lequel le fragment d'acide nucléique de référence comprenant une partie de la région 10q24.33 a été immobilisé.

3. Procédé de détection d'une délétion chromosomique selon la revendication 1 ou 2, dans lequel le fragment d'acide nucléique de référence comprenant une partie de la région 10q24.33 est un oligonucléotide, un ADNc, un ADN de BAC, un ADN de PAC, ou un ADN de YAC.

4. Procédé de détection d'une délétion chromosomique selon la revendication 3, dans lequel le fragment d'acide nucléique de référence comprenant une partie de la région 10q24.33 est l'ADN de BAC RP11-416N2.

5. Procédé de détection d'une délétion chromosomique selon l'une quelconque des revendications 1 à 4, dans lequel la délétion hémizygote dans la région 10q24.33 est détectée au moyen de la technique de puce à ADN, transfert de Southern, transfert de Northern, RT-PCR en temps réel, la technique FISH, ou la technique CGH.
